# EUROPÄISCHE PATENTSCHRIFT

(11) **EP 3 927 196 B1**
(45) Veröffentlichungstag und Bekanntmachung des Hinweises auf die Patenterteilung: **11.12.2024**
(21) Anmeldenummer: 20705931.2
(22) Anmeldetag: 14.02.2020
(51) Int. Cl.: A24F 40/10, A24F 40/70, A61M 15/06, B23K 1/06, B23K 20/10, H05B 3/04, H05B 3/44, A24F 40/42, A24F 40/44, A24F 40/46

(54) **VERDAMPFERVORRICHTUNG FÜR EINEN INHALATOR, VERBRAUCHSEINHEIT, INHALATOR UND HERSTELLUNGSVERFAHREN**
EVAPORATOR DEVICE FOR AN INHALER, CONSUMPTION UNIT, INHALER, AND PRODUCTION METHOD
DISPOSITIF VAPORISATEUR POUR INHALATEUR, UNITÉ DE CONSOMMATION, INHALATEUR ET PROCÉDÉ DE FABRICATION

(30) Priorität: 18.02.2019 DE 102019103987
(43) Veröffentlichungstag der Anmeldung: 29.12.2021
(73) Patentinhaber: Körber Technologies GmbH, 21033 Hamburg (DE)
(72) Erfinder: CORNILS, Lasse, 22605 Hamburg (DE); ROMMING, Niklas, 22085 Hamburg (DE); JAKLIN, Jan, 70736 Fellbach (DE); NIEBUHR, Gunnar, 21149 Hamburg (DE); ULLNER, Tim, 21039 Hamburg (DE); MÜLLER, Thomas, 20259 Hamburg (DE)
(74) Vertreter: Müller Verweyen
(86) Internationale Anmeldenummer: PCT/EP2020/053879
(87) Internationale Veröffentlichungsnummer: WO 2020/169467

(56) Entgegenhaltungen:
- EP-A1- 2 316 286
- EP-A1- 3 216 359
- WO-A1-2015/117701
- CN-A- 108 308 716
- CN-U- 207 926 944
- US-A1- 2016 345 629
- US-A1- 2018 064 170
- US-B2- 8 833 364

## Beschreibung

Die vorliegende Erfindung betrifft eine Verdampfervorrichtung für einen Inhalator, umfassend mindestens einen elektrischen Verdampfer zum Verdampfen von dem Verdampfer zugeführter Flüssigkeit, wobei die Flüssigkeit durch Kapillarkräfte von einer Einlassseite durch wenigstens einen Flüssigkeitskanal zu einer Auslassseite transportiert wird, wo verdampfte Flüssigkeit einem Luftstrom zugebbar ist, einen Träger, der den Verdampfer hält, und wenigstens eine den Verdampfer elektrisch kontaktierende elektrische Leitung. Die Erfindung betrifft auch eine Verbrauchseinheit, einen Inhalator und ein Herstellungsverfahren.

Herkömmliche Inhalatoren bzw. elektronische Zigarettenprodukte basieren auf der Docht-Wendel-Technologie. Durch Kapillarkräfte wird die Flüssigkeit aus einem Flüssigkeitsspeicher entlang eines Dochts so weit transportiert, bis die Flüssigkeit durch eine elektrisch beheizbare Wendel erhitzt und somit verdampft wird. Der Docht dient als flüssigkeitsleitende Verbindung zwischen dem Flüssigkeitsspeicher und der als Verdampfer dienenden Heizwendel.

in herkömmlichen Inhalatoren ist die elektrische Kontaktierung des Verdampfers durch die Docht-Wendel-Technologie simpel. Die Wendel muss lediglich mit einem elektrischen Strom beaufschlagt werden. Dadurch wird die Wendel erhitzt und Flüssigkeit kann verdampfen.

Ein Nachteil der Docht-Wendel-Technologie ist, dass eine mangelnde Versorgung mit Flüssigkeit zu einer lokalen Überhitzung führt, wodurch Schadstoffe entstehen können. Diesen sogenannte "Dry Puff" gilt es zu vermeiden. Zudem sind derartige Verdampfereinheiten fertigungsbedingt oft undicht, so dass Flüssigkeit auf ungewünschte Weise, zum Beispiel über die Luftzuführung und/oder Dampfabführung austreten kann.

Um die Probleme der Docht-Wendel-Technologie zu vermeiden, wird auf gattungsgemäße Verdampfer zurückgegriffen, die sich der in DE 10 2017 111 119 A1 offenbarten Technologie bedienen. Dabei wird die Flüssigkeit durch Kapillarkräfte von einer Einlassseite durch einen Flüssigkeitskanal zu einer Auslassseite transportiert, wo verdampfte Flüssigkeit als Dampf und/oder Aerosol einem Luftstrom zugebbar ist. Der Verdampfer ist elektrisch mit einer Energiequelle über eine elektrische Leitung verbindbar. Die Anbindung des Verdampfers an die elektrische Leitung ist im zitierten Stand der Technik jedoch nicht beschrieben.

US 2016/345629 A1 offenbart eine Verdampfervorrichtung nach dem Oberbegriff von Anspruch 1.

WO 2015/117701 A1, EP 2 316 286 A1, US 2018/064170 A1, US 8,833,364 B2 und CN 207 926 944 U offenbaren weitere Verdampfervorrichtungen für einen Inhalator.

Es ist die Aufgabe der Erfindung eine Verdampfervorrichtung mit einer zuverlässigen und effektiven elektrischen Verbindung zu dem Verdampfer bereitzustellen.

Die Aufgabe wird gelöst durch die Merkmale der unabhängigen Ansprüche.

Erfindungsgemäß wird vorgeschlagen, dass die elektrische Leitung einen planaren Kontaktbereich aufweist. Die Kontaktbereiche sind zur elektrisch leitendenden und stoffschlüssigen Verbindung vorgesehene Bereiche der elektrischen Leitung. Der planare Kontaktbereich der elektrischen Leitung verbessert die Geometrie der Verbindung, die zwischen der elektrischen Leitung und dem Verdampfer vorgesehen ist. Insbesondere ist der Kontaktbereich daher nicht punktförmig oder gekrümmt, wie bei einer elektrischen Leitung mit einem gekrümmten und insbesondere runden Querschnitt vorliegend. Daher kann in dem Kontaktbereich eine zuverlässige elektrische Verbindung zwischen der elektrischen Leitung und dem Verdampfer hergestellt werden. Ferner kann durch die planare Ausbildung des Kontaktbereichs eine vergrößerte Kontaktfläche zur Verwirklichung einer Verbindung und zur Stromübertragung erzielt werden. Dadurch können beispielsweise hohe Übergangswiderstände und damit einhergehende lokalisierte Temperaturspitzen vermieden werden.

Gemäß der Erfindung sind der Verdampfer und der planare Kontaktbereich der elektrischen Leitung miteinander stoffschlüssig und elektrisch leitend verbunden, um eine zuverlässige Verbindung zwischen der elektrischen Leitung und dem Verdampfer bereitzustellen. Zudem kann der stoffschlüssige Verbund den Verdampfer auf den Träger halten und/oder die Halterung des Verdampfers auf dem Träger unterstützen. Der aus der elektrischen Leitung und dem Verdampfer bestehende stoffschlüssige Verbund erlaubt eine präzise elektrische Kontaktierung, Anordnung und Halterung der Komponenten der Verdampfervorrichtung bei der Herstellung bzw. der Montage der Verdampfervorrichtung.

Vorzugsweise ist die äußere Form des Verdampfers blockförmig, und eine Seite des Verdampfers ist flächig mit der elektrischen Leitung verbunden, um die elektrische Verbindung zwischen der elektrischen Leitung und dem Verdampfer effektiv gestalten zu können und den Kontaktbereich effektiv zu nutzen. Vorteilhaft ist der Verdampfer an genau einer seiner Seiten, beispielsweise der Einlassseite oder der Auslassseite mit der elektrischen Leitung verbunden, um eine möglichst einfache Geometrie der Verdampfervorrichtung zu verwirklichen. Es ist jedoch auch möglich, dass beispielsweise zwei insbesondere einander gegenüberliegende Seiten des Verdampfers, beispielsweise jeweils senkrecht zu der Auslassseite oder der Einlassseite, mit jeweils mindestens einer elektrischen Leitung verbunden sind.

Bevorzugt hält der Träger die elektrische Leitung und/oder ist mit der elektrischen Leitung stoffschlüssig verbunden, um einen effektiven und kompakten Aufbau und eine effektive Herstellung der Verdampfervorrichtung zu begünstigen. Eine stoffschlüssige Verbindung der elektrischen Leitung mit dem Träger führt zu einem stoffschlüssigen Verbund umfassend den Träger, die elektrische Leitung und den Verdampfer.

Vorteilhaft weist der Verdampfer eine insbesondere rechteckige Grundfläche mit zwei gegenüberliegenden Randabschnitten auf, und jeder der gegenüberliegenden Randabschnitte ist mit einer elektrischen Leitung verbunden, um die Grundfläche des Verdampfers effektiv nutzen zu können und zwischen den Randabschnitten einen elektrischen Widerstand und somit eine Erwärmung und Verdampfung der Flüssigkeit hervorrufen zu können.

In einer bevorzugten Ausführungsform sind wenigstens zwei elektrische Leitungen vorgesehen und der Träger weist zwischen den elektrischen Leitungen eine Durchgangsöffnung auf. Der Verdampfer kann beispielsweise so angeordnet werden, dass die Durchgangsöffnung auf der Einlassseite des Verdampfers am Träger angeordnet ist. Die Durchgangsöffnung ermöglicht, dass eine Dochtstruktur die Einlassseite des Verdampfers zur Versorgung mit Flüssigkeit flächig kontaktieren kann. Die Durchgangsöffnung kann jedoch auch auf der Auslassseite des Verdampfers am Träger angeordnet sein, damit die vom Verdampfer verdampfte Flüssigkeit dem die Auslassseite überströmenden Luftstrom zugebbar ist.

Erfindungsgemäß umfasst der Träger zur Halterung des Verdampfers ein keramisches Substrat, um den Träger zur Halterung des Verdampfers thermisch stabil auszubilden und/oder gegebenenfalls den Verdampfer thermisch von dem Träger zu entkoppeln. Das keramische Substrat ist chemisch und mechanisch stabil gegenüber den im Betrieb des Verdampfers auftretenden Temperaturen von beispielsweise bis zu 300 °C und thermischen Lastwechseln, die beispielsweise ca. 200 - 2000 - mal im Lebenszyklus des Verdampfers auftreten. Der Träger steht im Kontakt zu der Flüssigkeit und/oder dem Aerosol bzw. Dampf und muss deshalb insbesondere bei den während der Verdampfung auftretenden Temperaturen lebensmitteltauglich bzw. biokompatibel sein, was durch das keramische Substrat begünstigt ist.

Vorteilhaft ist der Kontaktbereich aus Gold, um ein elektrisch gut leitenden Kontaktbereich bereitzustellen, der sich für eine stoffschlüssige und elektrisch leitende Verbindung zu dem Verdampfer eignet. Der Kontaktbereich aus Gold ist gegenüber der zu verdampfen Flüssigkeit bzw. dem Aerosol bzw. Dampf chemisch stabil und lässt sich zuverlässig durch an sich bekannte Verfahren herstellen und bearbeiten.

Erfindungsgemäß weist der Verdampfer eine Metallisierungsschicht auf, um mit elektrischen Kontaktflächen vorbereitet werden zu können, die in dem Kontaktbereich mit der elektrischen Leitung verbunden werden können. Der Verdampfer kann beispielsweise im Wesentlichen einen insbesondere dotierten Siliziumblock umfassen, der an der Oberfläche die Metallisierungsschicht aufweist. Die Metallisierungsschicht vereinfacht die elektrische und stoffschlüssige Kontaktierung der elektrischen Leitung mit dem Verdampfer.

In einer vorteilhaften Ausführungsform umfasst die Metallisierungsschicht Nickel, Gold und/oder Palladium, um für die stoffschlüssige Verbindung eine elektrisch leitende und mit bekannten Verfahren effektiv zu verarbeitende Oberfläche des Verdampfers vorbereiten zu können.

Erfindungsgemäß ist zwischen dem Verdampfer und der Metallisierungsschicht eine Grundierung angeordnet, beispielsweise ein seed-layer aus Aluminium, auf dem die vorteilhaft stromlos abgeschiedene Metallisierungsschicht aufgebracht ist.

Vorteilhaft weist die elektrische Verbindung zwischen dem Verdampfer und der elektrischen Leitung einen elektrischen Widerstand von 5 mΩ bis 20 mΩ auf, um die unerwünschte Umwandlung von elektrischer Energie in Wärme im Vergleich zu der zur Verdampfung erzeugten Wärme gering zu halten und den Verdampfer gleichzeitig mit genügend elektrischer Energie versorgen zu können.

Erfindungsgemäß ist die stoffschlüssige Verbindung wenigstens teilweise durch ein erstes gesintertes Material gebildet, um die Verbindung präzise und effektiv herstellen zu können. Durch Sintern der stoffschlüssigen Verbindung wird eine erhebliche Zeitersparnis beim Platzieren der Verdampfer auf dem Träger erzielt. In dieser Ausführungsform muss nicht jeder Kontakt des Verdampfers einzeln elektrisch angebunden (gebondet) werden. Eine mechanisch stabile und elektrisch leitfähige Verbindung kann durch das Sintern im Ofen parallel für eine Mehrzahl von Verdampfer gleichzeitig vorgenommen werden.

Erfindungsgemäß ist die elektrische Leitung wenigstens teilweise durch ein zweites gesintertes Material gebildet, um die elektrische Leitung präzise anordnen und effektiv herstellen zu können.

In einer vorteilhaften Ausführungsform umfasst die stoffschlüssige Verbindung zwischen dem Verdampfer und der elektrischen Leitung einen insbesondere elektrisch nicht leitfähigen Kleber, um eine stoffschlüssige Verbindung zwischen dem Verdampfer und der elektrischen Leitung herstellen zu können. Dabei kann die elektrische Verbindung zwischen der elektrischen Leitung und dem Verdampfer unabhängig von der stoffschlüssigen Verbindung gebildet und/oder angeordnet werden. Insbesondere kann die elektrische Verbindung in einem kleineren Bereich angeordnet werden als die stoffschlüssige Verbindung, um den Verdampfer gezielt beheizen und/oder elektrisch kontaktieren zu können. Gleichzeitig kann der Verdampfer flächig mit dem Träger verbunden werden.

Vorzugsweise weist der Verdampfer und/oder der Kontaktbereich Lötbumps auf, um eine präzise lokalisierte und effektiv auszuführende elektrische Verbindung zwischen dem Verdampfer und elektrischen Leitung herstellen zu können. Insbesondere können die Lötbumps zur vorteilhaften elektrischen Kontaktierung und Verarbeitung Goldbumps sein.

Vorteilhaft weist die elektrische Leitung einen thermischen Ausdehnungskoeffizienten auf, der um weniger als 10 % von dem thermischen Ausdehnungskoeffizienten des Verdampfers abweicht, um die Lebensdauer der Verdampfervorrichtung zu verbessern, da der Verdampfer im Betrieb bis zu 300 °C heiß wird und in seinem Lebenszyklus ca. 200 - 2000 - mal einen thermischen Lastwechsel zwischen Umgebungstemperatur und der Betriebstemperatur des Verdampfers erfährt. Der niedrige Unterschied von weniger als 10 % der thermischen Ausdehnungskoeffizienten führt zu nur einer niedrigen mechanischen Spannung im Bereich der stoffschlüssigen Verbindung zwischen der elektrischen Leitung und dem Verdampfer und belastet diese somit mechanisch nicht in einem kritischen Ausmaß.

Erfindungsgemäß umfasst eine Verbrauchseinheit für einen Inhalator die zuvor beschriebene Verdampfervorrichtung und einen Flüssigkeitsspeicher, wobei die Verdampfervorrichtung flüssigkeitsleitend mit dem Flüssigkeitsspeicher verbunden ist. Die Einlassseite des Verdampfers ist flüssigkeitsleitend mit dem Flüssigkeitsspeicher verbunden, beispielsweise über eine Dochtstruktur. Ein erfindungsgemäßer Inhalator umfasst die Verbrauchseinheit und eine Basiseinheit. Die stoffschlüssige und elektrisch leitendende Verbindung zwischen der elektrischen Leitung und dem Verdampfer führt dazu, dass die Verbrauchseinheit beziehungsweise der Inhalator effektiv zu montieren ist.

Ein Verfahren zur Herstellung der zuvor beschriebenen Verdampfervorrichtung, umfasst die folgenden Schritte: Bereitstellen des Verdampfers, des Trägers und der wenigstens einen elektrischen Leitung und stoffschlüssiges und elektrisch leitendes Verbinden des Verdampfers und der elektrischen Leitung in einem planaren Kontaktbereich des Verdampfers. Als Vorbereitung für die elektrisch leitende und stoffschlüssige Verbindung kann insbesondere bereits auf Wafer Basis optional eine entsprechende Metallisierung auf den Verdampfer aufgebracht werden. Als Gegenstück für den Verdampfer ist der Kontaktbereich der elektrischen Leitung vorgesehen.

Vorzugsweise umfasst das Verbinden ein Thermokompressionsverfahren. Vorteilhaft wird vor dem Verbinden der Kontaktbereich, der Träger und/oder der Verdampfer in vorgesehenen Bondingbereichen mit einer für eine Thermokompression geeigneten Sinterpaste belegt. Das Belegen der Kontaktbereiche mit einer Sinterpaste kann beispielsweise durch Schablonen, - Sieb,- und/oder Tampondruck erfolgen.

Vorteilhaft umfasst das Verbinden eine Anwendung von Ultraschall, um eine Reibschweißhitze zur thermomechanischen Verbindung mittels Thermokompression zwischen der elektrischen Leitung und dem Verdampfer zu begünstigen.

Bevorzugt umfasst das Verbinden Sintern, um eine einfache Vorbereitung für die stoffschlüssige Verbindung bereitstellen zu können. Als Vorbereitung für die Verbindung mittels Sintern kann bereits auf Wafer-Basis eine geeignete Metallisierung auf die Kontaktflächen der Verdampfer aufgebracht werden. Auch die elektrischen Leitungen selbst können durch Sintern hergestellt werden. Der stoffschlüssige Verbund kann aus einem ersten gesinterten Material bestehen und /oder die elektrische Leitung aus einem zweiten gesinterten Material. Es ist auch möglich, den Verbund und die elektrische Leitung in einem Arbeitsschritt und/oder aus dem gleichen gesinterten Material herzustellen. Vorteilhaft wird vor dem Verbinden der Kontaktbereich, der Träger und/oder der Verdampfer in vorgesehenen Sinterbereichen mit einer für das Sintern geeigneten Sinterpaste belegt. Für eine stabile Verbindung zwischen elektrischen Kontaktbereich und Verdampfer wird der Verbund entsprechend der Anforderung der Sinterpaste im Ofen gesintert. Vorteilhaft ist das Sintern drucklos, um insbesondere den Verdampfer mechanisch nicht zu belasten.

Vorzugsweise geschieht vor dem Verbinden ein Vereinzeln einer Mehrzahl gleichzeitig bearbeiteter Verdampfer, um möglichst effektiv eine Mehrzahl von Verdampfervorrichtungen herstellen zu können. Die Mehrzahl von Verdampfern kann in einem Wafer-Verbund vorliegen, wobei der Wafer-Verbund mehrere tausend Verdampfer ausbilden kann. Der Wafer-Verbund kann beispielsweise mittels einer Wafer-Säge vereinzelt werden und die Mehrzahl separater Verdampfer bereitstellen. Dafür kann je ein Verdampfer beispielsweise mittels eines Pick-Prozesses aus einem eine Mehrzahl von dem die Verdampfer bildenden Wafer-Verbund entnommen und beispielsweise mittels Flip-Chip-Montage mit der elektrischen Leitungen des Trägers elektrisch leitend und stoffschlüssig verbunden werden.

Bevorzugt kann vor dem Verbinden eine Mehrzahl von Lötbumps aufgebracht werden, um die elektrische Verbindung effektiv herstellen zu können.

Vorteilhaft umfasst das Verbinden ein Kleben mit einem insbesondere elektrisch nicht leitfähigen Kleber, um die stoffschlüssige Verbindung effektiv herstellen zu können. Dabei kann die Klebeverbindung an ausgewählten lokalen Flächen verwirklicht werden sein, während andere Flächen bewusst nicht mit dem elektrisch nicht leitenden Kleber benetzt also nicht verklebt werden und zur elektrischen Verbindung dienen.

Die Erfindung wird im Folgenden anhand bevorzugter Ausführungsformen unter Bezugnahme auf die beigefügten Figuren erläutert. Dabei zeigt
- Fig. 1: eine Explosionsdarstellung einer Verdampfervorrichtung und einer Trägervorrichtung;
- Fig. 2: eine perspektivische Darstellung einer Verdampfervorrichtung im montierten Zustand;
- Fig. 3: eine Explosionsdarstellung einer Verdampfervorrichtung und einer Trägervorrichtung;
- Fig. 4: eine perspektivische Darstellung einer Verdampfervorrichtung im montierten Zustand;
- Fig. 5: eine schematische Ansicht eines Inhalators; und
- Fig. 6: einen perspektivischen Schnitt durch eine Verdampfervorrichtung.

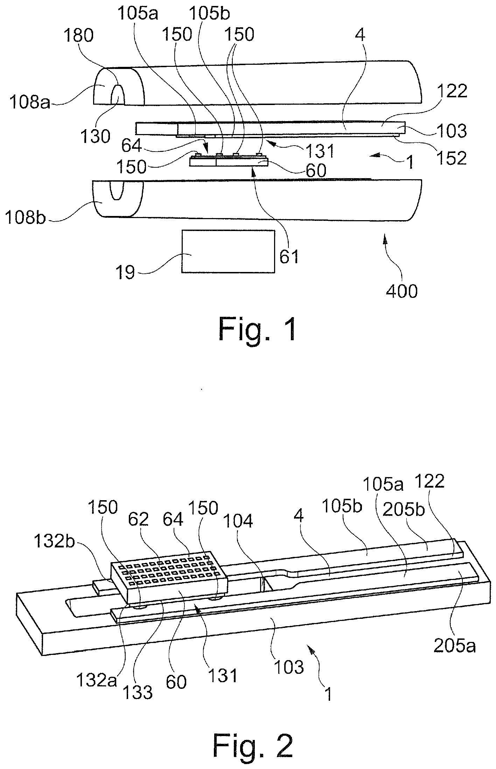

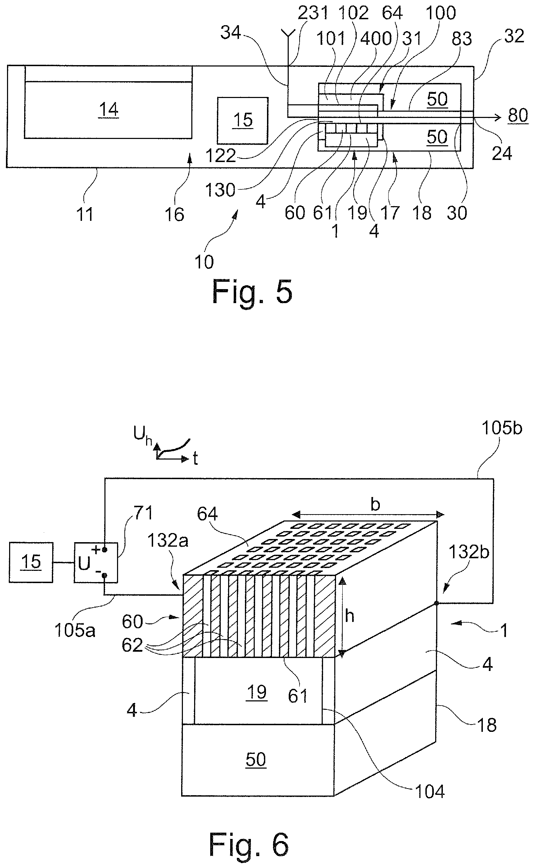

Figur 1 zeigt eine Explosionsdarstellung einer Verdampfervorrichtung 1 und einer Trägervorrichtung 400 für einen Inhalator 10. Die Verdampfervorrichtung 1 umfasst einen elektrischen Verdampfer 60 zum Verdampfen von dem Verdampfer 60 zugeführter Flüssigkeit 50 (siehe Figuren 5 und 6). Im montierten Zustand (siehe Figur 2) ist der Verdampfer 60 von einem Träger 4 gehalten. Der Verdampfer 60 weist eine Auslassseite 64 und eine Einlassseite 61 sowie sich dazwischen befindliche Flüssigkeitskanäle 62 auf und ist mit Bezug zu Figur 6 näher erläutert.

Der Träger 4 umfasst ein keramisches Substrat 103, siehe Figur 1. In dieser Ausführungsform ist das keramische Substrat 103 im wesentlichen plattenförmig. Beispielsweise kann das keramische Substrat 103 aus einer Zirkoniumoxidplatte oder einer Platte aus Keramik, insbesondere einer Niedertemperatur-Einbrand-Keramik, bestehen. Der Träger 4 weist wenigstens einen planaren Abschnitt auf, in dem ein planarer Kontaktbereich 131 ausgebildet ist. In dieser Ausführungsform ist der Träger 4 keramisch. Alternativ kann der Träger 4 aus einem hitzebeständigen Kunststoff bestehen und eine keramische Verdampferaufnahme umfassen.

Das Substrat 103 hat vorteilhaft einen zu dem Material, aus dem der Verdampfer 60 vorrangig besteht, beispielsweise Silizium, ähnlichen insbesondere um maximal 10 % abweichenden thermischen Ausdehnungskoeffizienten. Damit bleiben während des Betriebs des Verdampfers 60 mechanische Spannungen zwischen dem Träger 4 und dem Verdampfer 60 gering.

Vorteilhaft weist das Substrat 103 eine geringe thermische Leitfähigkeit und/oder eine geringe Wärmekapazität auf, um die zur Verdampfung vorgesehene Wärme nicht von dem Verdampfer 60 abzuleiten und/oder im Träger 4 zu speichern. Damit ist der Verdampfer 60 thermisch von dem Träger 4 und/oder von externen Bauteilen entkoppelt. Durch eine geringe Wärmeleitfähigkeit und/oder Wärmekapazität des Trägers 4 wird die vorteilhaft niedrige thermische Trägheit des Verdampfers 60 nicht beeinträchtigt. Dadurch kann der Verdampfer 60 zeitlich präzise und insbesondere schnell erhitzt werden und/oder sich wieder abkühlen, um eine hohe Aerosolqualität und eine präzise Einstellung der Aerosolmenge zu begünstigen.

Der Verdampfer 60 und der Träger 4 sind so angeordnet, dass der Träger 4 im montierten Zustand mit dem Verdampfer 60 an der Auslassseite 64 elektrisch und stoffschlüssig verbunden ist. Damit der Verdampfer 60 dem Luftstrom 34 die verdampfte Flüssigkeit zugeben kann, weist der Träger 4 eine nicht gezeigte Durchgangsöffnung 104 auf. Die Durchgangsöffnung 104 ist an der Auslassseite 64 des Verdampfers 60 angeordnet. Vorteilhaft ist die Durchgangsöffnung 104 mindestens so groß wie die Fläche des Verdampfers 60, die mit Flüssigkeitskanälen 62 versehen ist (siehe Figur 6).

Auf dem Träger 4 sind zwei elektrische Leitungen 105a, 105b vorgesehen, die mit dem Verdampfer 60 stoffschlüssig und elektrisch leitend verbunden werden, siehe Figur 1. Die elektrischen Leitungen 105a, 105b sind mit dem Träger 4 stoffschlüssig verbunden beziehungsweise aus den Träger 4 aufgebracht. Die elektrischen Leitungen 105a, 105b können beispielsweise Kupfer umfassen.

Die elektrischen Leitungen 105a, 105b weisen einen zu einer elektrisch leitenden und stoffschlüssigen Verbindung geeigneten Kontaktbereich 131 auf. Der Kontaktbereich 131 kann beispielsweise aus Gold bestehen, vorteilhaft aus einer Schicht Gold von vorzugsweise 5 - 50 µm, weiter vorzugsweise 8 - 20 µm, beispielsweise 10 µm.

Der Verbund aus Träger 4 und Verdampfer 60 wird beispielsweise zwischen die zwei Halbschalen 108a, 108b platziert, welche als Trägervorrichtung 400 beispielsweise die Aufgabe erfüllen, die Verdampfervorrichtung 1 und eine Dochtstruktur 19 relativ zu der Verdampfervorrichtung 1 zu halten, so dass eine gute Flüssigkeitsanbindung des Verdampfers 60 an ein externes Teil, beispielsweise einen Flüssigkeitsspeicher 18 besteht.

Die Halbschalen 108a, 108b stehen vorteilhaft aus einem Hochtemperatur-Kunststoff, beispielsweise PEEK, der bei den bei der Verdampfung auftretenden Temperaturen mechanisch und chemisch stabil bleibt.

In der oberen Schale 108a ist eine Ausnehmung 180 vorgesehen, so dass sich zwischen dem Träger 4 und der oberen Schale 108a ein Luftkanal 130 bildet. Der Luftkanal 130 wird im Betrieb des Inhalators 10 von einem Luftstrom 34 durchströmt. Der Luftstrom 34 überströmt die Auslassseite 64 des Verdampfers 60, der dem Luftstrom 34 verdampfte Flüssigkeit 50 als Dampf und/oder Aerosol zugibt.

In der unteren Schale 108b ist eine nicht gezeigte Öffnung vorgesehen, durch die sich die Dochtstruktur 19 erstreckt. Die Dochtstruktur 19 ist einerseits mit der Einlassseite 61 des Verdampfers 60 und andererseits mit einem Flüssigkeitsspeicher 18 flüssigkeitsleitend verbunden. Die Dochtstruktur 19 wird mit Bezug zu Figuren 5 und 6 näher erläutert.

Es ist auch möglich, nur eine elektrische Leitung 105a vorzusehen und den elektrischen Strom beispielsweise über die Halbschalen 108a, 108b oder ein Gehäuseteil abzuleiten (nicht gezeigt). Es ist auch möglich, dass eine weitere elektrische Kontaktierung der Leitungen 105a, 105b und/oder ein Nebenluftkanal 101 in einer der Schalen 108a, 108b vorgesehen ist. Die Trägervorrichtung 400 kann auch aus einer einstückigen Schale oder mehr als zwei Schalenteilen bestehen.

Wie in Figur 1 zu sehen ist, ist der Verdampfer 60 vorteilhaft ein insbesondere chipartiger Flachheizer. Der Verdampfer 60 ist blockförmig und die Auslassseite 64 ist im montierten Zustand flächig mit den elektrischen Leitungen 105a, 105b in dem planaren Kontaktbereich 131 verbunden.

Figur 2 zeigt eine perspektivische Darstellung einer Verdampfervorrichtung 1 im montierten Zustand. In dieser Ausführungsform ist die Einlassseite 61 des Verdampfers 60 mit den elektrischen Leitungen 105a, 105b flächig, elektrisch leitend und stoffschlüssig in dem Kontaktbereich 131 verbunden. Damit ist der Verdampfer 60 über den Kontaktbereich 131 mit dem Träger 4 elektrisch und mechanisch verbunden und gehalten.

Der Verdampfer 60 weist eine rechteckige Grundfläche mit zwei gegenüberliegenden Randabschnitten 132a, 132b auf. Der Verdampfer 60 ist jeweils in einem der Randabschnitte 132a, 132b mit einer der elektrischen Leitungen 105a, 105b verbunden. Die elektrischen Leitungen 105a, 105b verlaufen jeweils von dem Kontaktbereich 131 bis zu einem Endabschnitt 205a, 205b. An den Endabschnitten 205a, 205b ist die Verdampfervorrichtung 1 zur Versorgung des Verdampfers 60 mit elektrischer Energie von einem externen Teil kontaktierbar, beispielsweise einer Heizspannungsquelle 71. Die Endabschnitte 205a, 205b sind vorteilhaft zum Verlöten mit einer Kontaktierung des externen Teils oder zusammen mit dem Träger 4 als ein Steckverbinder ausgebildet.

Die Endabschnitte 205a, 205b sind vorteilhaft an einem sich stromaufwärts des Verdampfer 60 befindlichen Ende 122 des Trägers 4 zur Versorgung des Verdampfers 60 mit elektrischer Energie angeordnet. Das sich stromaufwärts des Verdampfers 60 befindliche Ende 122 ist das Ende, das durch die elektrische Kontaktierung zur mechanischen und/oder elektrischen Verbindung mit einem externen Teil, beispielsweise einem Basisteil 16 des Inhalators 10, eingerichtet ist. Die elektrischen Leitungen 105a, 105b verlaufen vorzugsweise parallel zum Luftkanal 130.

Zwischen den elektrischen Leitungen 105a, 105b weist der Träger eine Durchgangsöffnung 104 auf. Die Durchgangsöffnung 104 ist an der Einlassseite 61 des Verdampfers 60 angeordnet. Vorteilhaft ist die Durchgangsöffnung 104 zwischen den Kontaktbereichen 131 der elektrischen Leitungen 105a, 105b angeordnet. Durch die Durchgangsöffnung 104 kann sich eine Dochtstruktur 19 erstrecken, um die Einlassseite 61 des Verdampfers flüssigkeitsleitend zu kontaktieren. Die Durchgangsöffnung 104 weist vorteilhaft eine Form auf, die der Form der Dochtstruktur 19 entspricht.

In dem Kontaktbereich 131 sind optional auf den elektrischen Leitungen 105a, 105b mehrere Lötbumps 150 vorgesehen. Die Lötbumps 150 sind vorteilhaft aus Gold und können beispielsweise mittels Thermokompression eine elektrisch leitende Verbindung zwischen dem Verdampfer 60 und den elektrischen Leitungen 105a, 105b herstellen.

Um die Verbindung zwischen den elektrischen Leitungen 105a, 105b und dem Verdampfer 60 mechanisch und/oder thermisch zu verbessern, kann die Verbindung einen Kleber umfassen. Der Kleber kann insbesondere elektrisch nicht leitfähig sein, um die elektrisch leitende Verbindung nicht zu beeinträchtigen. Vorteilhaft weist der Kleber eine geringe Wärmeleitfähigkeit auf, um den Wärmeeintrag vom Verdampfer 60 in den Träger 4 zu verringern.

Zur Herstellung der in Figuren 1 und 2 gezeigten Verdampfervorrichtungen 1 werden der Träger 4 mit den elektrischen Leitungen 105a, 105b und der Verdampfer 60 bereitgestellt.

Der Verdampfer 60 kann in einem Wafer-Verbund vorliegen, aus dem eine Mehrzahl von Verdampfern 60 gefertigt wird. Die Mehrzahl von Verdampfern 60 wird beispielsweise durch verschiedene Ätzund Beschichtungsverfahren hergestellt, insbesondere indem der Wafer-Verbund durch Herstellung der Flüssigkeitskanäle 62 strukturiert wird.

Der Verdampfer 60 kann als Vorbereitung für die Verbindung mit den elektrischen Leitungen 105a, 105b mit einer Metallisierungsschicht 133 versehen werden. Die Metallisierungsschicht 133 kann direkt auf den Verdampfer 60 oder auf eine Grundierung, beispielsweise einen Seed-Layer aus Aluminium aufgebracht werden. Die Metallisierungsschicht 133 kann aus stromlos abgeschiedenen Nickel, Gold und/oder Palladium oder einem Materialgefüge daraus bestehen. Dies kann für eine Mehrzahl von Verdampfern 60, beispielsweise einem Wafer-Verbund, gleichzeitig passieren, um den Prozess zu parallelisieren.

Optional können die Lötbumps 150 auf die Oberfläche des Verdampfers 60 beziehungsweise auf dessen Metallisierungsschicht 133 und/oder auf die elektrischen Leitungen 105a, 105b beziehungsweise die Oberfläche des vorteilhaft keramischen Trägers 4 im Kontaktbereich 131 aufgebracht werden. Vorteilhaft wird dafür in dem Kontaktbereich 131 auf die elektrischen Leitungen 105a, 105b jeweils eine Schicht von Gold aufgetragen. Darauf können die Lötbumps 150 angeordnet werden.

Alternativ wird eine für die stoffschlüssige Verbindung geeignete Sinterpaste entweder auf die elektrischen Leitungen 105a, 105b des vorteilhaft keramischen Trägers 4 oder auf eine vorgesehene Kontaktfläche des Verdampfers 60 aufgedruckt und/oder aufgetragen.

Anschließend wird der Wafer-Verbund vereinzelt, womit eine Mehrzahl von Verdampfern 60 bereitgestellt wird. Die vereinzelten Verdampfer 60 werden beispielsweise mittels eines Pick-Prozesses aus dem Wafer-Verbund entnommen. Mittels Flip-Chip-Montage werden die Verdampfer 60 auf die elektrischen Leitungen 105a, 105b des vorteilhaft keramischen Trägers 4 mit Hilfe eines Thermokompressionsverfahren gebunden. Das Thermokompressionsverfahren kann durch die Anwendung von Ultraschall durch ein Einbringen von Reibschweißhitze verbessert werden. Optional kann der Verbund aus Verdampfer 60 und Träger 4 durch einen elektrisch nichtleitenden Kleber mechanisch stabilisiert werden.

Dabei können die Träger 4 in einem Trägerverbund vorliegen, auf den die Mehrzahl von Verdampfern 60 angeordnet und aufgebracht wird. Nach dem Bestücken und Verbinden der Verdampfer 60 mit den im Trägerverbund vorliegenden Trägern 4 können die somit entstehenden Verdampfervorrichtungen 1 vereinzelt werden.

Die Verdampfervorrichtungen 1 beziehungsweise die Verbünde aus Verdampfer 60 und Träger 4 werden dann zwischen den zwei Halbschalen 108a, 108b platziert. Die Trägerstruktur 400 kann dann in einen Verdampfereinsatz 100, eine Verbrauchseinheit 17 und/oder einen Inhalator 10 eingebaut werden.

In Figuren 1 und 2 ist der elektrisch leitende und stoffschlüssige Verbund aus Verdampfer 60 und Träger 4 durch ein Thermokompressionsverfahren unter Verwendung von Lötbumps 150 hergestellt.

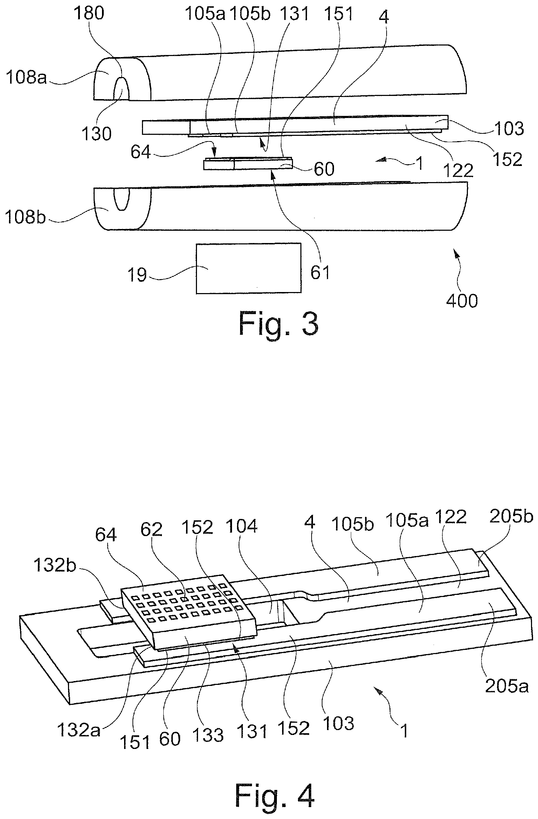

Figuren 3 und 4 zeigen eine Alternative zu der in Figuren 1 und 2 gezeigten Verbindung mit Hilfe der Lötbumps 150 unter Verwendung von gesinterten Material 151, 152. Die Figuren 3 und 4 werden in Hinblick auf die Unterschiede zu den Figuren 1 und 2 erläutert.

Figur 3 zeigt eine Explosionsdarstellung der Verdampfervorrichtung 1 und der Trägervorrichtung 400, wobei auf dem Verdampfer 60 ein erstes gesintertes Material 151 vorgesehen ist, um mit den elektrischen Leitungen 105a, 105b eine elektrisch leitende und stoffschlüssige Verbindung einzugehen. Figur 4 zeigt eine perspektivische Darstellung der Verdampfervorrichtung 1.

Der Kontaktbereich 131 der elektrischen Leitungen 105a, 105b auf dem Träger 4 und/oder die Kontaktfläche auf der Oberfläche des Verdampfers 60 ist zum insbesondere drucklosen Sintern eingerichtet, beispielsweise durch eine Schicht Gold.

Die elektrischen Leitungen 105a, 105b bestehen aus einem zweiten gesinterten Material 152. Das zweite gesinterte Material 152 wird auf das keramische Substrat 103 beziehungsweise den Träger 4 aufgebracht werden und bildet nach den Sintern die elektrischen Leitungen 105a, 105b aus. Durch Sintern werden das zweite gesinterte Material 152 und somit die elektrischen Leitungen 105a, 105b mit dem Träger 4 stoffschlüssig verbunden.

Es ist auch möglich, dass das erste gesinterte Material 151 aus dem gleichen Stoff besteht, wie das zweite gesinterte Material 152. In diesem Fall können in nur einem Sinterprozess die stoffschlüssig mit dem Träger 4 verbundenen elektrischen Leitungen 105a, 105b und gleichzeitig die Verbindung zwischen dem Verdampfer 60 und den elektrischen Leitungen 105a, 105b hergestellt werden.

Alternativ kann auch der Träger 4 mit bereits vorhandenen elektrischen Leitungen 105a, 105b bereitgestellt werden und nur die stoffschlüssige Verbindung zwischen Verdampfer 60 und den elektrischen Leitungen 105a, 105b kann aus dem zweiten gesinterten Material 152 gebildet sein.

Zur Herstellung der Verbindung mit dem ersten gesinterten Material 151 ist ein Aufbringen von Lötbumps entbehrlich. Dafür wird vorteilhaft eine für das Sintern geeignete Sinterpaste entweder auf die elektrischen Leitungen 105a, 105b des vorteilhaft keramischen Trägers 4 aufgedruckt bzw. aufgetragen. Nach dem Vereinzeln der Verdampfer 60 werden die Verdampfer 60 auf das erste Sintermaterial 151 auf den elektrischen Leitungen 105a, 105b platziert.

Alternativ kann das erste Sintermaterial 151 auf die vorgesehene Kontaktfläche des Verdampfers 60 aufgedruckt und/oder aufgetragen werden. Nach dem Vereinzeln der Verdampfer 60 werden die Verdampfer 60 auf den elektrischen Leitungen 105a, 105b platziert.

Für eine stabile Verbindung zwischen elektrischen Kontaktbereich 131 und Verdampfer 60 wird der Verbund aus Träger 4 und Verdampfer 60 mit dem ersten gesinterten Material 151 und optional mit dem zweiten gesinterten Material 152 für die elektrischen Leitungen 105a, 105b im Ofen gesintert.

Sowohl die in Figuren 1 und 2 sowie in Figuren 3 und 4 gezeigten Ausführungsbeispiele erlauben eine elektrische Kontaktierung des Verdampfers 60 bei gleichzeitig geringer Wärmeleitung vom Verdampfer 60 zum Träger 4. Die elektrische Verbindung zwischen dem Verdampfer 60 und der elektrischen Leitung 105a, 105b weist einen elektrischen Widerstand von 5 mΩ bis 20 mΩ auf. Durch eine Anpassung von Querschnitt und/oder Länge beziehungsweise Dicke der elektrischen Verbindung, also der Lötbumps 150 und/oder des ersten gesinterten Materials 151, ist die aus der Wärmeleitung vom Verdampfer 60 und der am elektrischen Widerstand zwischen der elektrischen Leitung 105a, 105b und dem Verdampfer 60 entstehende Wärme, die in den Träger 4 eingebracht wird, minimal.

Figur 5 zeigt schematisch einen Inhalator 10 beziehungsweise ein elektronisches Zigarettenprodukt. Der Inhalator 10 umfasst ein Gehäuse 11, in dem ein Luftstromkanal 30 zwischen mindestens einer Lufteinlassöffnung 231 und einer Luftauslassöffnung 24 an einem Mundende 32 des Inhalators 10 vorgesehen ist. Das Mundende 32 des Inhalators 10 bezeichnet dabei das Ende, an dem der Konsument zwecks Inhalation zieht und dadurch den Inhalator 10 mit einem Unterdruck beaufschlagt und einen Luftstrom 34 in dem Luftstromkanal 30 erzeugt.

Der Inhalator 10 besteht vorteilhaft aus einem Basisteil 16 und einer Verbrauchseinheit 17 beziehungsweise Verdampfer-Tank-Einheit, die die Verdampfervorrichtung 1 und den Flüssigkeitsspeicher 18 umfasst und insbesondere in Form einer auswechselbaren Kartusche ausgebildet ist. Die durch die Lufteinlassöffnung 231 angesaugte Luft wird in dem Luftstromkanal 30 zu der, oder durch die mindestens eine Verdampfervorrichtung 1 geleitet. Die Verdampfervorrichtung 1 ist mit dem Flüssigkeitsspeicher 18 verbunden oder verbindbar, in dem mindestens eine Flüssigkeit 50 gespeichert ist.

Die Verdampfervorrichtung 1 verdampft Flüssigkeit 50, die der Verdampfervorrichtung 1 aus dem Flüssigkeitsspeicher 18 vorteilhaft von einem Docht beziehungsweise einer Dochtstruktur 19 mittels Kapillarkräften zugeführt wird, und gibt die verdampfte Flüssigkeit als Aerosol/Dampf an einer Auslassseite 64 in den Luftstrom 34 zu.

An einer Einlassseite 61 des Verdampfers 60 ist vorteilhaft die poröse und/oder kapillare, flüssigkeitsleitende Dochtstruktur 19 angeordnet, wie schematisch in Figur 5 gezeigt. Es können eine Flüssigkeitsschnittstelle und/oder mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Die Dochtstruktur 19 kontaktiert die Einlassseite 61 des Verdampfers 60 vorteilhaft flächig und deckt sämtliche Flüssigkeitskanäle 62 des Verdampfers 60 einlassseitig ab. An der dem Verdampfer 60 gegenüberliegenden Seite ist die Dochtstruktur flüssigkeitsleitend mit dem Flüssigkeitsspeicher 18 verbunden. Der Flüssigkeitsspeicher 18 kann in seinen Abmessungen größer als die Dochtstruktur 19 sein. Die Dochtstruktur 19 kann beispielsweise in eine Öffnung eines Gehäuses des Flüssigkeitsspeichers 18 eingesetzt sein. Es kann auch eine Mehrzahl von Verdampfervorrichtungen 1 einem Flüssigkeitsspeicher 18 zugeordnet sein. Die Dochtstruktur 19 kann generell einteilig oder mehrteilig sein.

Die Dochtstruktur 19 besteht aus porösem und/oder kapillarem Material, das aufgrund von Kapillarkräften in der Lage ist, von dem Verdampfer 60 verdampfte Flüssigkeit in ausreichender Menge von dem Flüssigkeitsspeicher 18 zu dem Verdampfer 60 passiv nachzufördern, um ein Leerlaufen der Flüssigkeitskanäle 62 und sich daraus ergebende Probleme zu verhindern.

Die Dochtstruktur 19 besteht vorteilhaft aus einem elektrisch nichtleitenden Material, um eine unerwünschte Erwärmung von Flüssigkeit in der Dochtstruktur 19 durch Stromfluss zu vermeiden. Die Dochtstruktur 19 weist vorteilhaft eine geringe thermische Leitfähigkeit auf. Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm^3 und 10 mm^3, weiter vorzugsweise im Bereich zwischen 2 mm^3 und 8 mm^3, noch weiter vorzugsweise im Bereich zwischen 3 mm^3 und 7 mm^3 und beträgt beispielsweise 5 mm^3.

Falls die Dochtstruktur 19 aus einem elektrisch und/oder thermisch leitenden Material besteht, ist zwischen der Dochtstruktur 19 und dem Verdampfer 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Flüssigkeitskanälen 62 korrespondierenden Öffnungen vorgesehen.

Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-lonen-Akku, sein. Die Verbrauchseinheit 17 ist zwischen dem Energiespeicher 14 und dem Mundende 32 angeordnet. Die elektronische Steuerungsvorrichtung 15 umfasst mindestens eine digitale Datenverarbeitungseinrichtung, insbesondere Mikroprozessor und/oder Microcontroller, in dem Basisteil 16 (wie in Figur 5 gezeigt) und/oder in der Verbrauchseinheit 17 beziehungsweise einem Verdampfereinsatz 100.

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Inhalators 10 zieht, um zu inhalieren. In diesem Fall steuert die Steuerungsvorrichtung 15 die Verdampfervorrichtung 1 an, um Flüssigkeit 50 aus dem Flüssigkeitsspeicher 18 als Aerosol/Dampf in den Luftstrom 34 zuzugeben.

Die Verdampfervorrichtung 1 beziehungsweise der mindestens eine Verdampfer 60 ist in einem dem Mundende 32 abgewandten Teil der Verbrauchseinheit 17 angeordnet. Damit ist eine effektive elektrische Kopplung und Ansteuerung der Verdampfervorrichtung 1 möglich. Der Luftstrom 34 führt vorteilhaft durch einen axial durch den Flüssigkeitsspeicher 18 laufenden Luftstromkanal 30 zu der Luftauslassöffnung 24.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff, insbesondere Nikotin. Die angegebenen Bestandteile der Flüssigkeit 50 sind jedoch nicht zwingend. Insbesondere kann auf Aroma- und/oder Wirkstoffe, insbesondere Nikotin, verzichtet werden.

Die Verbrauchseinheit bzw. Kartusche 17 oder das Basisteil 16 umfasst vorteilhaft einen nichtflüchtigen Datenspeicher zum Speichern von die Verbrauchseinheit bzw. Kartusche 17 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft elektrisch mit der Steuereinrichtung 15 verbunden oder verbindbar.

In dem Inhalator 10 und/oder in einem externen Speicher, der in geeigneter und an sich bekannter Weise, zumindest zeitweilig, kommunikationstechnisch mit dem Inhalator 10 verbunden werden kann, könnten auch nutzerbezogene Daten, insbesondere über das Rauchverhalten, gespeichert und vorzugsweise auch zur Steuerung und Regelung des Inhalators genutzt werden.

Ein Verdampfereinsatz 100 ist zum Einsetzen in den Flüssigkeitsspeicher 18 vorgesehen. Der Flüssigkeitsspeicher weist dazu wenigstens eine Einsetzöffnung auf, in die der Verdampfereinsatz 100 eingesetzt, insbesondere eingeschoben werden kann. Der Verdampfereinsatz 100 umfasst ein Grundteil 83 zur Aufnahme der Trägervorrichtung 400, des Träger 4 und des Verdampfers 60. Das Grundteil 83 weist eine Mantelseite 31 auf, die den von dem Luftstrom 34 durchströmbaren Luftstromkanal 30 umschließt.

Das Grundteil 83 ist flüssigkeitsdicht und lässt keine Flüssigkeit 50 in den Innenraum des Verdampfereinsatzes 100 vordringen, um einen ungewollten Austritt von Flüssigkeit 50 aus dem Luftstromkanal 30 und/oder der Verbrauchseinheit 17 zu verhindern. Die Abdichtung des Verdampfereinsatzes 100 ist so ausgebildet, dass Flüssigkeit 50 nur den Weg durch die Dochtstruktur 19 und anschließend durch den Verdampfer 60 nehmen kann und im verdampften Zustand dem Luftstrom 34 zugegeben wird.

Der von der Trägervorrichtung 400 im Bereich des Verdampfers 60 gebildete Luftkanal 130 geht stromabwärts des Verdampfers 60 in den Luftstromkanal 30 über. Der Luftkanal 130 kann als der von Trägervorrichtung 400 gebildete Strömungsabschnitt des Luftstromkanals 130 aufgefasst werden.

Zusätzliche Kanäle, insbesondere wenigstens ein Nebenluftkanal 101, die stromabwärts vom Verdampfer 60 auf den Luftkanal 130 und/oder den Luftstromkanal 130 treffen, können für Durchmischung des Gas-/Aerosol-Gemisches mit Frischluft von einem Nebenluftstrom 102 sorgen und/oder Prozesse der Nachbehandlung und/oder der Rekondensation regeln.

Die Verdampfervorrichtung 1 gemäß Figur 6 umfasst einen blockförmigen, vorzugsweise monolithischer Heizkörper beziehungsweise Verdampfer 60 vorzugsweise aus einem elektrisch leitenden Material, insbesondere einem Halbleitermaterial vorzugsweise Silizium, und einen Träger 4 mit einer Durchgangsöffnung 104 zur flüssigkeitsleitenden Verbindung des Verdampfers 60 und einem Flüssigkeitsspeicher 18. Dafür ist vorteilhaft in der Durchgangsöffnung 104 eine Dochtstruktur 19 angeordnet.

Es ist nicht erforderlich, dass der gesamte Verdampfer 60 aus einem elektrisch leitenden Material besteht. Es kann beispielsweise ausreichen, dass die Oberfläche des Verdampfers 60 elektrisch leitend, beispielsweise metallisch, beschichtet oder vorzugsweise geeignet dotiert ist. In diesem Fall muss nicht die gesamte Oberfläche beschichtet sein, beispielsweise können metallische oder vorzugsweise nichtmetallische oder nichtmetallisch kaschierte metallische Leiterbahnen auf einem nichtleitenden beziehungsweise halbleitenden Grundkörper vorgesehen sein. Es ist auch nicht zwingend erforderlich, dass der gesamte Verdampfer 60 heizt; es kann beispielsweise ausreichen, wenn ein Abschnitt oder eine Heizschicht des Verdampfers 60 im Bereich der Austrittsseite 64 heizt.

Vorteilhaft weist der Verdampfer 60 wenigstens auf einer Kontaktfläche, die zur elektrischen Kontaktierung vorgesehen ist, eine Metallisierungsschicht 133 auf.

Der Verdampfer 60 ist mit einer Mehrzahl von Mikrokanälen beziehungsweise Flüssigkeitskanälen 62 versehen, die eine Einlassseite 61 des Verdampfer 60 mit einer Auslassseite 64 des Verdampfer 60 flüssigkeitsleitend verbinden. Die Einlassseite 61 ist über eine nicht in Figur 6 gezeigte Dochtstruktur 19 flüssigkeitsleitend mit dem Flüssigkeitsspeicher 18 verbunden. Die Dochtstruktur 19 dient zur passiven Förderung von Flüssigkeit aus dem Flüssigkeitsspeicher 18 zum Verdampfer 60 mittels Kapillarkräften.

Der mittlere Durchmesser der Flüssigkeitskanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Flüssigkeitskanal 62 eindringende Flüssigkeit durch den Flüssigkeitskanal 62 nach oben steigt, bis der Flüssigkeitskanal 62 mit Flüssigkeit gefüllt ist. Das Volumenverhältnis von Flüssigkeitskanälen 62 zu Verdampfer 60, das als Porosität des Verdampfers 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Flüssigkeitskanälen 62 versehenen Flächen des Verdampfer 60 liegen beispielsweise im Bereich zwischen 0,5 mm und 3 mm, vorzugsweise zwischen 0.5 mm und 1 mm. Die Abmessungen der mit Flüssigkeitskanälen 62 versehenen Flächen des Verdampfers 60 können beispielsweise betragen: 0,95 mm × 1,75 mm oder 1,9 mm × 1,75 mm oder 1,9 mm × 0,75 mm. Die Kantenlängen des Verdampfers 60 können beispielsweise im Bereich zwischen 0,5 mm und 5 mm, vorzugsweise im Bereich zwischen 0,75 mm und 4 mm, weiter vorzugsweise im Bereich zwischen 1 mm und 3 mm liegen. Die Fläche des Verdampfers 60 (chip size) kann beispielsweise 1 mm × 3 mm, 2mm × 2 mm oder 2 mm × 3 mm betragen.

Die Breite b des Verdampfer 60 (siehe Figur 6) liegt vorzugsweise im Bereich zwischen 1 mm und 5 mm, weiter vorzugsweise im Bereich zwischen 2 mm und 4 mm, und beträgt beispielsweise 3 mm.

Die Höhe h des Verdampfer 60 (siehe Figur 6) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm. Auch noch kleinere Verdampfer 60 können gefertigt, vorgesehen und funktionsgerecht betrieben werden.

Die Anzahl der Flüssigkeitskanäle 62 liegt vorzugsweise im Bereich zwischen vier und 1000. Auf diese Weise lässt sich der Wärmeeintrag in die Flüssigkeitskanäle 62 optimieren und eine gesicherte hohe Verdampfungsleistung sowie eine ausreichend große Dampfaustrittsfläche realisieren.

Die Flüssigkeitskanäle 62 sind in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Flüssigkeitskanäle 62 mit gesichert hoher Verdampfungsleistung realisieren.

Der Querschnitt der Flüssigkeitskanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Flüssigkeitskanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von Verdampfer 60 in die Flüssigkeitskanäle 62 realisieren.

Der Abstand zweier Flüssigkeitskanäle 62 beträgt vorzugsweise mindestens das 1,3-fache des lichten Durchmessers eines Flüssigkeitskanals 62, wobei der Abstand auf die Mittelachsen der beiden Flüssigkeitskanäle 62 bezogen ist. Der Abstand kann bevorzugt das 1,5- bis 5-fache, weiter bevorzugt das 2- bis 4-fache des lichten Durchmessers eines Flüssigkeitskanals 62 betragen. Auf diese Weise lässt sich ein optimaler Wärmeeintrag in den Verdampfer 60 und eine ausreichend stabile Anordnung und Wandstärke der Flüssigkeitskanäle 62 realisieren.

Aufgrund der vorbeschriebenen Merkmale kann der Verdampfer 60 auch als Volumenheizer bezeichnet werden.

Die Verdampfervorrichtung 1 weist eine vorzugsweise von der Steuerungsvorrichtung 15 steuerbare Heizspannungsquelle 71 auf, die über elektrische Leitungen 105a, 105b in einem Kontaktbereich 131 (nicht in Figur 6 gezeigt) an gegenüberliegenden Randabschnitten 132a, 132b des Verdampfers 60 mit diesem verbunden ist, so dass eine von der Heizspannungsquelle 71 erzeugte elektrische Spannung Uh zu einem Stromfluss durch den Verdampfer 60 führt. Aufgrund des Ohm'schen Widerstands des elektrisch leitenden Verdampfers 60 führt der Stromfluss zu einer Erhitzung des Verdampfers 60 und daher zu einer Verdampfung von in den Flüssigkeitskanälen 62 enthaltener Flüssigkeit. Auf diese Weise erzeugter Dampf/Aerosol 6 entweicht zur Auslassseite 64 aus den Flüssigkeitskanälen 62 und wird dem Luftstrom 34 beigemischt. Genauer steuert bei Feststellung eines durch Ziehen des Konsumenten verursachten Luftstroms 34 durch den Luftstromkanal 30 die Steuerungsvorrichtung 15 die Heizspannungsquelle 71 an, wobei durch spontane Erhitzung die in den Flüssigkeitskanälen 62 befindliche Flüssigkeit in Form von Dampf/Aerosol aus den Flüssigkeitskanälen 62 getrieben wird.

Eine elektronische beziehungsweise elektrische Anbindung des Verdampfers 60 an die elektrischen Leitungen 105a, 105b ist mit Bezug zu Figuren 1 bis 4 erläutert.

Vorzugsweise ist in dem Datenspeicher des Inhalators 10 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Verdampfers 60, und damit auch die Temperatur der kapillaren Flüssigkeitskanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100 °C und 400 °C, weiter bevorzugt zwischen 150 °C und 350 °C, noch weiter bevorzugt zwischen 190 °C und 290 °C.

Der Verdampfer 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise 750 µm, noch weiter vorzugsweise kleiner oder gleich 500 µm aufweist. Oberflächen des Verdampfers 60 können vorteilhaft hydrophil sein. Die Auslassseite 64 des Verdampfers 60 kann vorteilhaft mikrostrukturiert sein bzw. Mikroausnehmungen (micro grooves) aufweisen.

Die Verdampfervorrichtung 1 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfervorrichtung 1 hinsichtlich der Flüssigkeits-/Dampfmenge pro Zug, d.h. je Zugdauer von 1 s bis 3 s, einstellbar sein.

Im Folgenden wird beispielhaft der Ablauf des Verdampfungsvorgangs erläutert.

In einem Ausgangszustand ist die Spannungsquelle 71 beziehungsweise der Energiespeicher 14 für den Heizvorgang ausgeschaltet.

Zum Verdampfen von Flüssigkeit 50 wird die Spannungsquelle 14, 71 für den Verdampfer 60 aktiviert. Die Spannung Uh wird dabei so eingestellt, dass die Verdampfungstemperatur in dem Verdampfer 60 und somit in den Flüssigkeitskanälen 62 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der Flüssigkeitskanäle 62 entspricht oder damit in Zusammenhang steht, wird die Heizspannungsquelle 71 deaktiviert. Da die Liquideigenschaften und -menge vorteilhaft exakt bekannt sind und der Verdampfer 60 einen messbaren temperaturabhängigen Widerstand aufweist, kann dieser Zeitpunkt sehr genau bestimmt bzw. gesteuert werden.

Nach Abschluss des Heizvorgangs sind die Flüssigkeitskanäle 62 überwiegend oder vollständig entleert. Die Heizspannung 71 wird dann so lange ausgeschaltet gehalten, bis mittels Nachförderung von Flüssigkeit durch die Dochtstruktur 19 die Flüssigkeitskanäle 62 wieder aufgefüllt sind. Sobald dies der Fall ist, kann der nächste Heizzyklus durch einschalten der Heizspannung 71 begonnen werden.

Die von der Heizspannungsquelle 71 erzeugte Ansteuerfrequenz des Verdampfers 60 liegt im Allgemeinen vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Die Frequenz und der Tastgrad der Heizspannung Uh für den Verdampfer 60 sind vorteilhaft an die Eigenschwingung bzw. Eigenfrequenz der Blasenschwingungen während der Blasensiedung angepasst. Vorteilhaft kann die Periodendauer 1/f der Heizspannung daher im Bereich zwischen 5 ms und 50 ms, weiter vorteilhaft zwischen 10 ms und 40 ms, noch weiter vorteilhaft zwischen 15 ms und 30 ms liegen und beispielsweise 20 ms betragen. Je nach Zusammensetzung der verdampften Flüssigkeit 50 können andere als die genannten Frequenzen optimal an die Eigenschwingung bzw. Eigenfrequenz der Blasenschwingungen angepasst sein.

Des Weiteren hat sich gezeigt, dass der durch die Heizspannung Uh erzeugten maximale Heizstrom vorzugsweise nicht mehr als 7 A, weiter vorzugsweise nicht mehr als 6,5 A, noch weiter vorzugsweise nicht mehr als 6 A betragen und optimalerweise im Bereich zwischen 4 A und 6 A liegen sollten, um konzentrierten Dampf bei Vermeidung von Überhitzung zu gewährleisten.

Die Förderrate der Dochtstruktur 19 ist wiederum optimal an die an die Verdampfungsrate des Verdampfers 60 angepasst, so dass jederzeit ausreichend Flüssigkeit 50 nachgefördert werden kann und ein Leerlaufen des Bereichs vor dem Verdampfer 60 vermieden wird.

Die Verdampfervorrichtung 1 ist vorzugsweise auf der Grundlage von MEMS-Technologie, insbesondere aus Silizium, gefertigt und daher vorteilhaft ein Mikro-Elektro-Mechanisches System.

Vorgeschlagen wird nach dem zuvor Gesagten vorteilhaft ein Schichtaufbau bestehend aus einem vorteilhaft mindestens auf der Einlassseite 61 planaren Verdampfer 60 auf Si-Basis und einer oder mehrerer darunter liegender Kapillarstrukturen 19 mit vorteilhaft unterschiedlicher Porengröße. Die direkt an der Einlassseite 61 des Verdampfers 60 angeordnete Dochtstruktur 19 verhindert die Bildung von Blasen an der Einlassseite 61 des Verdampfers 60, da Gasblasen eine weitere Förderwirkung unterbinden und gleichzeitig zu einer (lokalen) Überhitzung des Verdampfers 60 aufgrund fehlender Kühlung durch nachströmendes Liquid führen.

## Patentansprüche

1. Verdampfervorrichtung (1) für einen Inhalator (10), umfassend
- mindestens einen elektrischen Verdampfer (60) zum Verdampfen von dem Verdampfer (60) zugeführter Flüssigkeit (50), wobei die Flüssigkeit (50) durch Kapillarkräfte von einer Einlassseite (61) durch wenigstens einen Flüssigkeitskanal (62) zu einer Auslassseite (64) transportiert wird, wo verdampfte Flüssigkeit (50) einem Luftstrom (34) zugebbar ist,
- einen Träger (4), der den Verdampfer (60) hält, und
- wenigstens eine den Verdampfer (60) elektrisch kontaktierende elektrische Leitung (105a, 105b), wobei
- die elektrische Leitung (105a, 105b) einen planaren Kontaktbereich (131) aufweist, und
- der Verdampfer (60) und der planare Kontaktbereich (131) der elektrischen Leitung (105a, 105b) miteinander stoffschlüssig und elektrisch leitend verbunden sind, **dadurch gekennzeichnet, dass**
- die stoffschlüssige Verbindung wenigstens teilweise durch ein erstes gesintertes Material (151) gebildet ist,
- der Träger (4) ein keramisches Substrat (103) aufweist,
- der Verdampfer (60) eine Metallisierungsschicht (133) aufweist,
- zwischen dem Verdampfer (60) und der Metallisierungsschicht (133) eine Grundierung angeordnet ist, und
- die elektrische Leitung (105) wenigstens teilweise durch ein zweites gesintertes Material (152) gebildet ist.

2. Verdampfervorrichtung (1) nach Anspruch 1, **dadurch gekennzeichnet, dass**
- die äußere Form des Verdampfers (60) blockförmig ist und
- eine Seite (61, 64) des Verdampfers (60) flächig mit der elektrischen Leitung (105a, 105b) verbunden ist.

3. Verdampfervorrichtung (1) nach einem der vorangehenden Ansprüche, **dadurch gekennzeichnet, dass**
- der Verdampfer (60) eine insbesondere rechteckige Grundfläche mit zwei gegenüberliegenden Randabschnitten (132a, 132b) aufweist und jeder der gegenüberliegenden Randabschnitte (132a, 132b) mit einer elektrischen Leitung (105a, 105b) verbunden ist.

4. Verdampfervorrichtung (1) nach einem der vorangehenden Ansprüche, **dadurch gekennzeichnet, dass**
- der Träger (4) die elektrische Leitung (105a, 105b) hält und/oder mit der elektrischen Leitung (105a, 105b) stoffschlüssig verbunden ist.

5. Verdampfervorrichtung (1) nach einem der vorangehenden Ansprüche, **dadurch gekennzeichnet, dass**
- wenigstens zwei elektrische Leitungen (105a, 105b) vorgesehen sind und
- der Träger (4) zwischen den elektrischen Leitungen (105a, 105b) eine Durchgangsöffnung (104) aufweist.

6. Verdampfervorrichtung (1) nach einem der vorangehenden Ansprüche, **dadurch gekennzeichnet, dass**
- der Kontaktbereich (131) aus Gold ist.

7. Verdampfervorrichtung (1) nach einem der vorangehenden Ansprüche, **dadurch gekennzeichnet, dass**
- die Metallisierungsschicht (133) Nickel, Gold und/oder Palladium umfasst.

8. Verdampfervorrichtung (1) nach einem der vorangehenden Ansprüche, **dadurch gekennzeichnet, dass**
- die elektrische Verbindung zwischen dem Verdampfer (60) und der elektrischen Leitung (105) einen elektrischen Widerstand von 5 mΩ bis 20 mΩ aufweist.

9. Verdampfervorrichtung (1) nach einem der vorangehenden Ansprüche, **dadurch gekennzeichnet, dass**
- die stoffschlüssige Verbindung zwischen dem Verdampfer (60) und der elektrischen Leitung (105) einen insbesondere elektrisch nicht leitfähigen Kleber umfasst.

10. Verdampfervorrichtung (1) nach einem der vorangehenden Ansprüche, **dadurch gekennzeichnet, dass**
- der Verdampfer (60) und/oder der Kontaktbereich (131) Lötbumps (150) aufweist.

11. Verdampfervorrichtung (1) nach einem der vorangehenden Ansprüche, **dadurch gekennzeichnet, dass**
- die elektrische Leitung (105a, 105b) einen thermischen Ausdehnungskoeffizienten aufweist, der um weniger als 10 % von dem thermischen Ausdehnungskoeffizienten des Verdampfers (60) abweicht.

12. Verbrauchseinheit (17) für einen Inhalator (10), umfassend
- eine Verdampfervorrichtung (1) nach einem der vorangehenden Ansprüche und
- einen Flüssigkeitsspeicher (18), wobei
- die Verdampfervorrichtung (1) flüssigkeitsleitend mit dem Flüssigkeitsspeicher (18) verbunden ist.

13. Inhalator (10), umfassend
- eine Verbrauchseinheit (17) nach Anspruch 12 und
- eine Basiseinheit (16).

14. Verfahren zur Herstellung einer Verdampfervorrichtung (1) nach einem der vorangehenden Ansprüche 1 bis 11, umfassend die folgenden Schritte:
- Bereitstellen des Verdampfers (60), des Trägers (4) und der wenigstens einen elektrischen Leitung (105a, 105b) und
- stoffschlüssiges und elektrisch leitendes Verbinden des Verdampfers (60) und der elektrischen Leitung (105a, 105b) in einem planaren Kontaktbereich des Verdampfers (60).

15. Verfahren nach Anspruch 14, **dadurch gekennzeichnet, dass**
- das Verbinden ein Thermokompressionsverfahren umfasst.

16. Verfahren nach Anspruch 14 oder 15, **dadurch gekennzeichnet, dass**
- das Verbinden eine Anwendung von Ultraschall umfasst

17. Verfahren nach einem der vorangehenden Ansprüche 14 bis 16, **dadurch gekennzeichnet, dass**
- das Verbinden ein insbesondere druckloses Sintern umfasst.

18. Verfahren nach einem der vorangehenden Ansprüche 14 bis 17, **dadurch gekennzeichnet, dass**
- vor dem Verbinden eine Mehrzahl gleichzeitig bearbeiteter Verdampfer (60) vereinzelt werden.

19. Verfahren nach einem der vorangehenden Ansprüche 14 bis 18, **dadurch gekennzeichnet, dass**
- vor dem Verbinden eine Mehrzahl von Lötbumps (150) aufgebracht werden.

20. Verfahren nach einem der vorangehenden Ansprüche 14 bis 19, **dadurch gekennzeichnet, dass**
- das Verbinden ein Kleben mit einem insbesondere nicht leitfähigen Kleber umfasst.

## Claims

1. Vaporizer device (1) for an inhaler (10), comprising
- at least one electric vaporizer (60) for vaporizing liquid (50) fed to the vaporizer (60), wherein the liquid (50) is transported by capillary forces from an inlet side (61) through at least one liquid channel (62) to an outlet side (64), where vaporized liquid (50) can be added to an air flow (34),
- a carrier (4) retaining the vaporizer (60), and
- at least one electrical line (105a, 105b) electrically contacting the vaporizer (60), wherein
- the electrical line (105a, 105b) comprises a planar contact area (131), and
- the vaporizer (60) and the planar contact area (131) of the electrical line (105a, 105b) are bonded materially and electrically conductively to each other, **characterized in that**
- the material bond is at least partially formed by a first sintered material (151),
- the carrier (4) comprises a ceramic substrate (103),
- the vaporizer (60) comprises a metallization layer (133),
- a primer is arranged between the vaporizer (60) and the metallization layer (133), and
- the electrical line (105) is at least partially formed by a second sintered material (152).

2. Vaporizer device (1) according to claim 1, **characterized in that**
- the outer shape of the vaporizer (60) is block-shaped, and
- one side (61, 64) of the vaporizer (60) is bonded in a planar manner to the electrical line (105a, 105b).

3. Vaporizer device (1) according to one of the preceding claims,
**characterized in that**
- the vaporizer (60) comprises a base surface, in particular a rectangular base surface, with two opposing edge sections (132a, 132b), and each of the opposing edge sections (132a, 132b) is bonded to an electrical line (105a, 105b).

4. Vaporizer device (1) according to one of the preceding claims,
**characterized in that**
- the carrier (4) retains the electrical line (105a, 105b) and/or is materially bonded to the electrical line (105a, 105b).

5. Vaporizer device (1) according to one of the preceding claims,
**characterized in that**
- at least two electrical lines (105a, 105b) are provided, and
- the carrier (4) comprises a passage opening (104) between the electrical lines (105a, 105b).

6. Vaporizer device (1) according to one of the preceding claims,
**characterized in that**
- the contact area (131) is made of gold.

7. Vaporizer device (1) according to claim 8, **characterized in that**
- the metallization layer (133) comprises nickel, gold and/or palladium.

8. Vaporizer device (1) according to one of the preceding claims,
**characterized in that**
- the electrical bond between the vaporizer (60) and the electrical line (105) comprises an electrical resistance of 5 mΩ to 20 mΩ.

9. Vaporizer device (1) according to one of the preceding claims,
**characterized in that**
- the material bond between the vaporizer (60) and the electrical line (105) comprises an adhesive, in particular an electrically non-conductive adhesive.

10. Vaporizer device (1) according to one of the preceding claims,
**characterized in that**
- the vaporizer (60) and/or the contact area (131) comprises solder bumps (150).

11. Vaporizer device (1) according to one of the preceding claims,
**characterized in that**
- the electrical line (105a, 105b) comprises a coefficient of thermal expansion that differs by less than 10% from the coefficient of thermal expansion of the vaporizer (60).

12. Consumption unit (17) for an inhaler (10), comprising
- a vaporizer device (1) according to any of the preceding claims, and
- a liquid reservoir (18), wherein
- the vaporizer device (1) is connected to the liquid reservoir (18) in a liquid-conducting manner.

13. Inhaler (10), comprising
- a consumption unit (17) according to claim 12, and
- a base unit (16).

14. Method for manufacturing a vaporizer device (1) according to any of the preceding claims 1 to 11, comprising the following steps:
- Providing the vaporizer (60), the carrier (4), and the at least one electrical line (105a, 105b), and
- materially and electrically conductively bonding the vaporizer (60) and the electrical line (105a, 105b) in a planar contact area of the vaporizer (60).

15. Method according to claim 14, **characterized in that**
- the bonding comprises a thermocompression process.

16. Method according to claim 14 or 15, **characterized in that**
- the bonding comprises an application of ultrasound.

17. Method according to any of the preceding claims 14 to 16,
**characterized in that**
- the bonding comprises sintering in particular without pressure.

18. Method according to any one of the preceding claims 14 to 17,
**characterized in that**
- a plurality of simultaneously processed vaporizers (60) are separated prior to bonding.

19. Method according to any one of the preceding claims 14 to 18,
**characterized in that**
- a plurality of solder bumps (150) are applied prior to bonding.

20. Method according to one of the preceding claims 14 to 19,
**characterized in that**
- the bonding comprises an adhesive bonding with an in particular non-conductive adhesive.

## Revendications

1. Dispositif vaporisateur (1) pour un inhalateur (10), comprenant
- au moins un vaporisateur (60) électrique destiné à la vaporisation de liquide (50) acheminé dans le vaporisateur (60), le liquide (50) étant transporté par des forces capillaires depuis un côté entrée (61), en passant par au moins un canal à liquide (62), jusqu'à un côté sortie (64) où du liquide (50) vaporisé peut être ajouté à un flux d'air (34),
- un support (4) qui maintient le vaporisateur (60), et
- au moins une ligne électrique (105a, 105b) mise en contact électrique avec le vaporisateur (60), dans lequel
- la ligne électrique (105a, 105b) présente une zone de contact (131) planaire, et
- le vaporisateur (60) et la zone de contact (131) planaire de la ligne électrique (105a, 105b) sont reliés l'un à l'autre par liaison de matière et de manière électroconductrice, **caractérisé en ce que**
- la liaison par liaison de matière est formée au moins en partie par un premier matériau fritté (151),
- le support (4) présente un substrat (103) en céramique,
- le vaporisateur (60) présente une couche de métallisation (133),
- une couche d'apprêt est agencée entre le vaporisateur (60) et la couche de métallisation (133), et
- la ligne électrique (105) est formée au moins en partie par un second matériau fritté (152).

2. Dispositif vaporisateur (1) selon la revendication 1, **caractérisé en ce que**
- la forme extérieure du vaporisateur (60) est en forme de bloc et
- un côté (61, 64) du vaporisateur (60) est relié par sa surface à la ligne électrique (105a, 105b).

3. Dispositif vaporisateur (1) selon l'une des revendications précédentes, **caractérisé en ce que**
- le vaporisateur (60) présente une surface de base en particulier rectangulaire avec deux parties de bord (132a, 132b) opposées et chacune des parties de bord (132a, 132b) opposées est reliée à une ligne électrique (105a, 105b).

4. Dispositif vaporisateur (1) selon l'une des revendications précédentes, **caractérisé en ce que**
- le support (4) maintient la ligne électrique (105a, 105b) et/ou est relié par liaison de matière à la ligne électrique (105a, 105b).

5. Dispositif vaporisateur (1) selon l'une des revendications précédentes, **caractérisé en ce que**
- au moins deux lignes électriques (105a, 105b) sont prévues et
- le support (4) présente une ouverture de passage (104) entre les lignes électriques (105a, 105b).

6. Dispositif vaporisateur (1) selon l'une des revendications précédentes, **caractérisé en ce que**
- la zone de contact (131) est en or.

7. Dispositif vaporisateur (1) selon l'une des revendications précédentes, **caractérisé en ce que**
- la couche de métallisation (133) comprend du nickel, de l'or et/ou du palladium.

8. Dispositif vaporisateur (1) selon l'une des revendications précédentes, **caractérisé en ce que**
- la liaison électrique entre le vaporisateur (60) et la ligne électrique (105) présente une résistance électrique de 5 mΩ à 20 mΩ.

9. Dispositif vaporisateur (1) selon l'une des revendications précédentes, **caractérisé en ce que**
- la liaison par liaison de matière entre le vaporisateur (60) et la ligne électrique (105) comprend une colle en particulier non électroconductrice.

10. Dispositif vaporisateur (1) selon l'une des revendications précédentes, **caractérisé en ce que**
- le vaporisateur (60) et/ou la zone de contact (131) présente des perles de soudure (150).

11. Dispositif vaporisateur (1) selon l'une des revendications précédentes, **caractérisé en ce que**
- la ligne électrique (105a, 105b) présente un coefficient de dilatation thermique qui diffère de moins de 10 % du coefficient de dilatation thermique du vaporisateur (60).

12. Unité de consommation (17) pour un inhalateur (10), comprenant
- un dispositif vaporisateur (1) selon l'une des revendications précédentes et
- un réservoir de liquide (18), dans lequel
- le dispositif vaporisateur (1) est relié au réservoir de liquide (18) de manière à permettre un passage de liquide.

13. Inhalateur (10), comprenant
- une unité de consommation (17) selon la revendication 12 et
- une unité de base (16).

14. Procédé de fabrication d'un dispositif vaporisateur (1) selon l'une des revendications précédentes 1 à 11, comprenant les étapes suivantes :
- mise à disposition du vaporisateur (60), du support (4) et de l'au moins une ligne électrique (105a, 105b) et
- liaison par liaison de matière et de manière électroconductrice du vaporisateur (60) et de la ligne électrique (105a, 105b) dans une zone de contact planaire du vaporisateur (60).

15. Procédé selon la revendication 14, **caractérisé en ce que**
- la liaison comprend un procédé de thermocompression.

16. Procédé selon la revendication 14 ou 15, **caractérisé en ce que**
- la liaison comprend l'utilisation d'ultrasons.

17. Procédé selon l'une des revendications précédentes 14 à 16, **caractérisé en ce que**
- la liaison comprend un frittage en particulier sans pression.

18. Procédé selon l'une des revendications précédentes 14 à 17, **caractérisé en ce que**
- avant la liaison, une pluralité de vaporisateurs (60) confectionnés simultanément sont séparés.

19. Procédé selon l'une des revendications précédentes 14 à 18, **caractérisé en ce que**
- avant la liaison, une pluralité de perles de soudure (150) sont appliquées.

20. Procédé selon l'une des revendications précédentes 14 à 19, **caractérisé en ce que**
- la liaison comprend un collage avec une colle en particulier non conductrice.
